# FASCICULE DE BREVET EUROPEEN

(11) **EP 0 317 453 B1**
(45) Date de publication et mention de la délivrance du brevet: **03.02.1993**
(21) Numéro de dépôt: 88420384.5
(22) Date de dépôt: 16.11.1988
(51) Int. Cl.: A61K 35/78

(54) **Compositions médicamenteuses à base de flavonoides et de saponines extraits de chrysanthellum, leur procédé de préparation et applications thérapeutiques**
Medizinische Zusammensetzungen, gegründet auf Flavonoiden und Saponinen, gewonnen aus Chrysanthellum, Verfahren zu ihrer Herstellung und therapeutische Anwendungen
Medicinal compositions based on flavonoids and saponins extracted from chrysanthellum, process for their manufacture and therapeutical uses

(30) Priorité: 19.11.1987 FR 8716668
(43) Date de publication de la demande: 24.05.1989
(73) Titulaire: IPHYM SA Société Anonyme dite :, F-01700 Beynost (FR)
(72) Inventeur: Guillot, Bernard, F-69570 Dardilly (FR)
(74) Mandataire: Maureau, Philippe

(56) Documents cités:
- FR-A- 2 096 902
- FR-A- 2 276 060
- FR-M- 979
- DIALOP 80091583 MEDLINE; M. BECCHI et al.: "Structure of a new saponin: chrysantellin A from Chrysanthellum procumbens Rich", & EUR. J. BIOCHEM, 1979, 102(1), 11-20

## Description

La présente invention concerne des compositions médicamenteuses à base de flavonoïdes et de saponines extraits de chrysanthellum ainsi qu'un procédé de préparation de ces extraits et l'utilisation de ces flavonoïdes et saponines pour obtenir un médicament destiné au traitement de la lithiase cystinique, des artérites, des insuffisances veineuses et des dislipidémies.

Le chrysanthellum est une plante sub-tropicale appartenant à la famille des Hélianthées.

Cette plante se rencontre en Afrique Centrale et en Amérique du Sud et certaines de ses variétés : Americanum, Procumbens, Indicum et Afro-Americanum ont été utilisées sous forme d'extraits aqueux ou hydro-alcoolique, dans des buts médicamenteux.

C'est ainsi que le BSM-FR 979 et le FR-A-2 096 902 décrivent des médicaments obtenus à partir d'extraits aqueux ou hydro-alcooliques de Chrysanthellum Americanum Vatke ; ces médicaments sont destinés au traitement des troubles intestinaux associés ou non à des troubles hépatiques.

Il faut noter que les extraits de Chrysanthellum proposés se présentent sous la forme soit de solutés instables, soit d'une masse brunâtre compacte et hygroscopique, de composition incomplètement définie et comportant à côté de substances pharmacologiquement actives non isolées une forte proportion de ballast inerte.

Cette composition ainsi définie est bien évidemment un obstacle à l'emploi de cet extrait comme médicament dans des conditions de posologie précises et reproductibles.

FR-A-2 276 060 décrit, par ailleurs, une méthode de purification plus poussée d'extraits de Chrysanthellum qui permet d'obtenir des extraits pulvérulents de composition mieux définie. Cette méthode exige au moins vingt lavages dans l'acétate d'éthyle et conduit à l'obtention d'un extrait très concentré en composés polyphénoliques, mais peu ou mal défini quant à la nature des composés qui accompagnent ces polyphénols

Les médicaments obtenus à partir de ces extraits sont donc essentiellement constitués de dérivés flavonoïdes, notamment O-déhydroxylés et ils présentent une activité sur la perméabilité capillaire.

Au cours de ses recherches, l'Inventeur a réussi à déterminer de façon surprenante qu'il était possible d'associer aux dérivés flavonoïdes extraits du Chrysanthellum des saponines, en quantité définie, également extraites de cette plante pour obtenir des compositions médicamenteuses nouvelles présentant des propriétés pharmacologiques différentes de celles des médicaments à base d'extraits de Chrysanthellum connus jusqu'alors.

Les compositions médicamenteuses selon l'invention sont donc caractérisées en ce que leur principe actif est essentiellement constitué de l'association de flavonoïdes et de saponines extraits du Chrysanthellum.

Le rapport moyen entre les flavonoïdes et les saponines est de 2/1.

Selon un mode de réalisation préféré de l'invention, le principe actif des compositions médicamenteuses renferme, en outre, des dérivés de l'acide caféique et de l'acide chlorogénique.

L'invention concerne également un procédé de préparation de l'extrait de Chrysanthellum prévu pour constituer le principe actif des compositions médicamenteuses selon l'invention.

Ce procédé va maintenant être décrit.

Le matériel végétal utilisé au départ est choisi parmi les variétés suivantes de Chrysanthellum : Chrysanthellum americanum et sa variété procumbens et Chrysanthellum indicum, sous-espèce afro-americanum.

On soumet l'ensemble de la plante fraîche ou sèche, à un broyage mécanique jusqu'à obtention d'une poudre grossière.

On fait ensuite macérer cette poudre pendant 10 à 24 heures à température ambiante dans de l'eau ou dans un solvant polaire tel que l'alcool de titre supérieur à 30°, par exemple de l'alcool à 60° ou à 96°.

L'extraction du principe actif peut ensuite se faire à froid, par macération ou lixiviation dans un solvant quelconque : eau, alcool éthylique, alcool méthylique, alcool butylique.

La durée de l'extraction est, bien entendu, fonction du temps d'humectation du matériel végétal et de son épuisement progressif.

C'est ainsi que, par exemple, une extraction de 200 Kg de poudre de plante à température ambiante d'environ 20°C dans de l'alcool éthylique à 60°C a donné de bons résultats. L'extraction peut également se faire à chaud dans de l'éthanol ou dans un autre solvant alcoolique, mais le titre du solvant alcoolique est alors supérieur à 50°.

L'extraction comporte, de préférence, une étape de macération suivie d'une percolation itérative.

L'étape suivante de purification de l'extrait obtenu est tout à fait décisive pour l'obtention du produit selon l'invention. Il convient, en effet, que cette purification n'élimine pas les constituants doués d'activité que l'on désire maintenir dans le produit, et tout spécialement les flavonoïdes et les saponines.

L'Inventeur a pu déterminer que l'on atteignait ce but en limitant la purification à un maximum de deux lavages à l'aide d'un solvant organique non polaire tel que le dichlorométhane.

Ces lavages sont effectués, de préférence, dans les conditions suivantes : dans des cuves ou bacs inox ou verre, à un volume d'extrait primaire est ajouté un volume d'un des solvants suivants : dichlorométhane, acétate d'éthyle, isopropanol, tertiobutylméthyléther ou toluène. Les deux phases sont soumises à une forte agitation pendant 1 à 2 heures. Après décantation, l'opération est renouvelée une fois, à température de + 10 a + 25°C.

Cette opération de lavage peut également être réalisée en présence de sels de relargage tels que sulfate de sodium ou sulfate d'ammonium. A titre d'exemple la phase organique de l'extrait reçoit 10 pour cent (P/V) de sulfate de sodium sous forte agitation continue. Après 2 heures de contact à + 20°C, l'extrait est centrifugé ou décanté puis séché rapidement.

On obtient, en fin d'opération, une poudre adaptée aux transformations galéniques usuelles. Cette poudre renferme des flavonoïdes et des saponines dans le rapport moyen de 2/1.

Des examens effectués en résonance magnétique nucléaire, en spectrométrie de masse et en chromatographie liquide haute pression ont permis de déterminer que l'extrait obtenu présente la composition suivante :
- Saponines :: chrysantelline A et B
dérivés de acide échinocystique et caulophyllogénine,
- Flavonoïdes :: - glucosyl 7 isookanine
- glucosyl 7 eriodictyol
- glucosyl 7 lutéoline
- mareïne
- maritiméïne
- Apigénine :: apigénine

Acides caféiques, chlorogénique et isochlorogé.

On a procédé, par ailleurs, sur l'extrait ainsi obtenu à une série d'essais toxicologiques permettant de déterminer :
- la toxicité à six mois chez le rat,
- la toxicité de la reproduction/fertilité.

On a également effectué des tests de tératologie ainsi que des tests de mutagénicité.

Les résultats de ces essais et tests ont permis de déterminer que le traitement chronique de 4 lots de 40 rats pendant 26 semaines est sans effet toxique à la dose moyenne de 125 DTH, que les tests de tératologie chez le rat et chez le lapin n'ont signalé aucun effet embryotoxique et tératogène aux doses de 250 à 360 DTH et que l'étude de la reproduction chez le rat ne montre aucune action délétère.

Les tests pharmacologiques montrent que l'extrait selon l'invention possède des propriétés anti-oedémateuses, telles que celles données dans le test à la carragénine sur la patte du lapin, et des effets protecteurs vis-à-vis des thrombi veineux chez le lapin.

Enfin, de nombreux essais cliniques ont permis de déterminer que l'extrait de Chrysanthellum selon l'invention constituait le principe actif de compositions médicamenteuses présentant une activité thérapeutique tout à fait surprenante comparativement aux autres substances médicamenteuses classiques à base d'extraits de Chrysanthellum.

C'est ainsi que les substances médicamenteuses renfermant l'extrait de Chrysanthellum selon l'invention sont spécialement actives dans le traitement d'une lithiase très difficile à soigner, la lithiase cystinique.

Des essais, in vitro d'abord, sur le pouvoir anticristallisant de l'extrait de Chrysanthellum et des essais in vivo ensuite sur plusieurs malades cystiniques permettent de proposer l'extrait dans le traitement de cette maladie.

Des malades chroniques, sous traitement pendant dix-huit mois, n'ont eu aucune rechute et leur cristallurie cystinique est restée normale.

Des évaluations effectuées en milieu hospitalier auprès de quatre-vingt hommes et cent trente six femmes ont permis d'étudier les propriétés des compositions médicamenteuses selon l'invention dans le traitement des artérites et de l'insuffisance veineuse des membres inférieurs.

Dans le cas de l'artérite, l'association naturelle des flavonoïdes et des saponines dans l'extrait selon l'invention intervient, non seulement, sur la microcirculation et la dynamique capillaire, mais aussi sur la rhéologie, l'agrégation plaquettaire et erythrocytaire et la réaction inflammatoire et oedémateuse, alors que l'extrait décrit dans FR-A-2 270 060 ne traitait que les vasculopathies induites par fragilité capillaire.

Dans le cas de l'insuffisance veineuse, cette même association intervient, outre sur la dynamique capillaire, sur la continence valvulaire.

L'extrait de Chrysanthellum selon l'invention trouve également une utilisation spécialement intéressante pour obtenir un médicament destiné au traitement des dislipidémies et spécialement de l'hypercholestérolémie et de l'hyperlipidémie. Une population, dûment définie et sous surveillance biologique et diététique, voit son taux moyen de cholestérol abaissé de 17 % et son taux moyen de trigycérides de 56 % après un à deux mois de traitement à raison de 300 mg d'extrait par jour.

Les compositions médicamenteuses selon l'invention associées à un support pharmaceutiquement acceptable peuvent être administrées par voie locale ou générale. Par voie générale il faut entendre essentiellement la voie orale (comprimés, dragées, gélules, capsules, poudres, sirops, solutés).

## Revendications

1. Compositions médicamenteuses, caractérisées en ce que leur principe actif est essentiellement constitué de l'association de flavonoïdes et de saponines extraits du Chrysanthellum.

2. Compositions selon la revendication 1, caractérisées en ce que le rapport moyen entre les flavonoïdes et les saponines est de 2/1.

3. Compositions selon la revendication 1 et la revendication 2, caractérisées en ce que le principe actif des compositions médicamenteuses renferme, en outre, des dérivés de l'acide caféique et de l'acide chlorogénique.

4. Compositions selon l'une des revendications 1 à 3, caractérisées en ce que les flavonoïdes sont au moins au nombre de six et choisis parmi les flavonoïdes - glucosyl 7 isookanine - glucosyl 7 eriodictyol - glucosyl 7 lutéoline - mareïne - maritimeïne - apigénine - acides caféique, chlorogénique et isochlorogénique.

5. Compositions selon l'une des revendications 1 à 3, caractérisées en ce que les saponines sont au moins au nombre de deux et choisis parmi les chrysantelline A et B dérivé de acide échinocystique et caulophyllogénine.

6. Compositions selon la revendication 3, caractérisées en ce que les dérivés de l'acide caféique et de l'acide chlorogénique et isochlorogénique sont conservés dans l'extrait.

7. Procédé pour l'obtention de l'extrait constituant le principe actif de la composition médicamenteuse selon l'une quelconque des revendications 1 à 6, caractérisé en ce qu'il consiste à soumettre une plante choisie parmi les Chrysanthellum dit americanum et sa variété procumbens et les Chrysanthellum indicum, sous-espèce afro-américanum à un traitement de broyage suivi d'une extraction par un solvant polaire puis à soumettre l'extrait obtenu à un maximum de deux lavages à l'aide d'un solvant non polaire.

8. Procédé selon la revendication 7, caractérisé en ce que l'étape d'extraction se fait par macération ou lixiviation dans un alcool de titre supérieur à 30°, ou dans l'eau, à froid.

9. Procédé selon la revendication 7, caractérisé en ce que l'étape d'extraction se fait par macération suivie d'une percolation itérative dans un alcool de titre supérieur à 50°, à chaud.

10. Utilisation de l'association des dérivés flavonoïdes et saponines extraits du Chrysanthellum pour l'obtention d'un médicament destiné au traitement de la lithiase cystinique.

11. Utilisation de l'association de dérivés flavonoïdes et saponines extraits du Chrysanthellum pour l'obtention d'un médicament destine au traitement des artérites et de l'insuffisance veineuse des membres inférieurs.

## Claims

1. Medicinal compositions, characterised in that their active principle consists essentially of a combination of flavonoids and saponins which are extracted from Chrysanthellum.

2. Compositions according to Claim 1, characterised in that the average ratio of flavonoids to saponins is 2:1.

3. Compositions according to Claim 1 and Claim 2, characterised in that the active principle of the medicinal compositions contains, in addition, derivatives of caffeic acid and of chlorogenic acid.

4. Compositions according to one of Claims 1 to 3, characterised in that the flavonoids number at least six and are chosen from the flavonoids - isookanin 7-glucoside - eriodictyol 7-glucoside - luteolin 7-glucoside - marein - maritimein - apigenin - caffeic, chlorogenic and isochlorogenic acids.

5. Compositions according to one of Claims 1 to 3, characterised in that the saponins number at least two and are chosen from chrysantellin A and B derived from echinocystic acid and caulophyllogenin.

6. Compositions according to Claim 3, characterised in that the derivatives of caffeic acid and of chlorogenic and isochlorogenic acid are preserved in the extract.

7. Process for obtaining the extract constituting the active principle of the medicinal composition according to any one of Claims 1 to 6, characterised in that it consists in subjecting a plant chosen from Chrysanthellum termed americanum and its variety procumbens and Chrysanthellum indicum, subspecies afro-americanum to a grinding treatment followed by extraction with a polar solvent, and then in subjecting the extract obtained to a maximum of two washes using a non-polar solvent.

8. Process according to Claim 7, characterised in that the extraction step is carried out by maceration or leaching in an alcohol of titre above 30° strength, or in water, in the cold state.

9. Process according to Claim 7, characterised in that the extraction step is carried out by maceration followed by a repetitive percolation in an alcohol of titre above 50° strength, in the heated state.

10. Use of the combination of flavonoid derivatives and saponins extracted from Chrysanthellum for obtaining a medicinal product intended for the treatment of cystine lithiasis.

11. Use of the combination of flavonoid derivatives and saponins extracted from Chrysanthellum for the production of a medicinal product intended for the treatment of arteritis and venous insufficiency of the lower limbs.

## Patentansprüche

1. Medikamentöse Zusammensetzungen, dadurch gekennzeichnet, daß deren Wirkstoff im wesentlichen durch die Kombination von aus Chrysanthellum gewonnenen Flavonoiden und Saponinen gebildet ist.

2. Zusammensetzungen nach Anspruch 1, dadurch gekennzeichnet, daß das mittlere Verhältnis zwischen den Flavonoiden und den Saponinen 2/1 ist.

3. Zusammensetzungen nach dem Anspruch 1 und dem Anspruch 2 dadurch gekennzeichnet, daß der Wirkstoff der medikentösen Zusammensetzung außerdem Derivate der Kaffeesäure und der Chlorogensäure einschließt.

4. Zusammensetzungen nach einem der Ansprüche 1 bis 3, dadurch gekennzeichnet, daß zumindest sechs Flavonoide vorhanden sind, und zwar ausgewählt aus den Flavonoiden Isookanin-7-glucosid, Eriodictyol-7-glucosid, Luteolin-7-glucosid, Marein, Maritimein, Apigenin, Kaffeesäure, Chlorogensäure, und Isochlorogensäure.

5. Zusammensetzungen nach einem der Ansprüche 1 bis 3, dadurch gekennzeichnet, daß zumindest zwei Saponine vorhanden sind, und zwar ausgewählt aus den Chrysanthellinen A und B abgeleitet von der Echinocystinsäure und der Caulophyllogeninsäure.

6. Zusammensetzungen nach Anspruch 3, dadurch gekennzeichnet, daß die Derivate der Kaffeesäure sowie der Chlorogen- und der Isochlorogensäure im Extrakt enthalten sind.

7. Verfahren zum Erlangen des Extraktes, der den Wirkstoff der medikamentösen Zusammensetzung nach einem der Ansprüche 1 bis 6 bildet, dadurch gekennzeichnet, daß es darin besteht, eine Pflanze, ausgewählt aus Chrysanthellum, nämlich Americanum, deren Procumbens-Varianten, Chrysanthellum Indicum, Unterart Afro-Americanum einer Zerkleinerungsbehandlung zu unterwerfen, gefolgt von einer Extraktion mit einem polaren Lösungsmittel, wobei anschließend der erhaltene Extrakt höchstens zwei Waschungen mit Hilfe eines nicht polaren Lösungsmittels unterworfen wird.

8. Verfahren nach Anspruch 7, dadurch gekennzeichnet, daß der Extraktionsschritt durch Mazeration oder Auslaugen in der Kälte in einem mehr als 30 %-igem Alkohol oder in Wasser erfolgt.

9. Verfahren nach Anspruch 7, dadurch gekennzeichnet, daß der Extraktionsschritt durch Mazeration gefolgt von einer mehrfachen Perkolation in der Wärme mit einem mehr als 50 %-igen Alkohol gebildet wird.

10. Verwendung der Kombination von aus Chrysanthellum gewonnenen Flavonoid- und Saponinderivaten zur Herstellung eines Medikamentes zur Behandlung von cystinischer Lithiase.

11. Verwendung der Kombination von aus Chrysanthellum gewonnenen Flavonoid- und Saponinderivaten zur Herstellung eines Medikamentes zur Behandlung von Arterienentzündungen und venösen Insuffizienzen der unteren Gliedmaßen.
